# EUROPEAN PATENT SPECIFICATION

(11) **EP 1 982 637 B1**
(45) Date of publication and mention of the grant of the patent: **18.03.2015**
(21) Application number: 08007330.7
(22) Date of filing: 14.04.2008
(51) Int. Cl.: A61B 1/005, G02B 23/24, A61B 19/00, B25J 13/02, A61B 1/01, A61B 17/00

(54) **Endoscopic operation assisting device**
Hilfsvorrichtung für Endoskopbetrieb
Système d'assistance pour une opération endoscopique

(30) Priority: 19.04.2007 US 788313
(43) Date of publication of application: 22.10.2008
(73) Proprietor: Olympus Medical Systems Corp., Tokyo 151-0072 (JP)
(72) Inventor: Murakami, Kazushi, Tokyo 151-0072 (JP); Onuki, Yoshio, Tokyo 151-0072 (JP); Komiya, Takaaki, Tokyo 151-0072 (JP); Ichikawa, Hiroaki, Tokyo 151-0072 (JP); Hashimoto, Tatsutoshi, Tokyo 151-0072 (JP); Honda, Kazuki, Tokyo 151-0072 (JP); Kura, Yasuhito, Tokyo 151-0072 (JP)
(74) Representative: von Hellfeld, Axel

(56) References cited:
- EP-A- 1 704 822
- EP-A- 1 782 744
- JP-A- 6 304 126
- JP-A- 63 267 326
- JP-A- 2003 140 055
- JP-A- 2005 168 531
- US-A- 5 976 075
- US-A1- 2002 103 418
- US-A1- 2003 018 237
- US-A1- 2003 212 308
- US-A1- 2004 133 075
- US-B1- 6 981 945

## Description

### BACKGROUND OF THE INVENTION

### 1. Field of the Invention

The present invention relates to an endoscopic operation assisting device attachable and detachable with respect to an insertion section of an endoscope, and particularly with respect to a flexible tube portion forming the insertion section.

### 2. Description of Related Art

In recent years, an endoscope has been widely used in the medical field. In general, the endoscope includes an elongated insertion section, and the insertion section is provided with a bendable bending portion at a distal end side thereof. A proximal end side of the insertion section is provided with an operation section, and the operation section is provided with a knob, a switch, and so forth for performing various operations of endoscope functions.

In the endoscope, the insertion section is inserted into a body cavity of a subject body to enable the observation of an organ, or, if necessary, a treatment instrument is inserted into the body cavity through a treatment instrument channel to enable a variety of treatments.

In the introduction of the treatment instrument into the body cavity through the treatment instrument channel, a surgeon manually inserts the treatment instrument having a length of no less than two meters into the channel. The manual insertion operation takes effort and requires attentiveness. Further, various operations of the treatment instrument performed after the insertion operation are extremely complicated.

To solve the problem, Japanese Unexamined Patent Application Publication No. 2000-207, for example, discloses an endoscopic treatment instrument inserting and withdrawing device. In addition to the function of inserting and withdrawing a treatment instrument with respect to a treatment instrument channel of an endoscope, the endoscopic treatment instrument inserting and withdrawing device includes treatment instrument operation means for operating a treatment portion provided at the distal end of the treatment instrument. A variety of operations of the treatment instrument inserting and withdrawing device can be performed with a foot switch. In the endoscopic treatment instrument inserting and withdrawing device, therefore, the insertion operation of the treatment instrument and the operation of the treatment portion can be performed with the foot switch, with the hands resting on the endoscope.

However, the surgeon performs examination and treatment while observing an endoscopic image photographed by the endoscope and displayed on a display device. Therefore, if the device is configured such that the foot switch is provided with a plurality of operation switches, to select a desired operation switch, the surgeon needs to avert his eyes from the display device to check the desired switch out of the plurality of switches located near his foot. Further, the insertion operation of the treatment instrument and the operation of the treatment portion are performed with the foot. Thus, it is difficult to perform a delicate operation.

Further, International Patent Application Publication No. WO2004-021868 discloses a system for controlling an endoscopic accessory provided to an external portion of an endoscope. The system is configured to include the endoscopic accessory disposed on an external portion of the distal end of the endoscope, a control sheath, and a control handle. The control handle can be slidably attached to an outer surface of an endoscope shaft so that the control handle and an endoscope handle can be grasped by one of the hands.

Furthermore, Japanese Unexamined Patent Application Publication No. 2006-263474 discloses a catheter grasping device which measures the insertion force during a medical procedure. The catheter grasping device includes a first handle member and a second handle member, which can be disposed to a catheter. The first handle member and the second handle member are openably and closably connected by a hinge assembly. When the catheter grasping device is not held in a closed state by a surgeon, the hinge assembly biases the first handle member and the second handle member to bring the catheter grasping device into an open state.

In the catheter grasping device, therefore, the disposed position of the catheter grasping device with respect to the catheter can be easily changed by shifting from the closed state to the open state. Further, each of the first handle member and the second handle member is provided with an elastic member. Thus, if the first handle member and the second handle member are brought into the closed state to surround the catheter, the respective elastic members come in contact with the catheter. Thereby, it is possible to press the catheter into a body cavity while grasping the catheter with the catheter grasping device.

However, in the catheter grasping device disclosed in the Japanese Unexamined Patent Application Publication No. 2006-263474, an outer surface of the handle is a flat and monotonous surface, so that the hand and fingertips do not fit the handle well and it has been difficult to shift the catheter grasping device from the open state to the closed state. In addition, also when the catheter is advanced or retreat in the closed state, it has been difficult to perform advancing or retreating operation because the hand slips on the monotonous surface.

On the other hand, in the control handle of the system for controlling the endoscopic accessory disclosed in the International Patent Application Publication No. WO2004-021868, the operation lever for operating a medical instrument used together with the endoscope is disposed on the same line as the insertion section to be grasped, so that there is a danger that the operation lever is accidentally operated during the operation of the insertion section. Furthermore, in the control handle, the operation lever is disposed on generally the same surface as the outer circumferential surface of the handle, so that there is a problem in operability, because it is difficult for the operator to move the thumb to operate the lever as he or she wishes when trying to operate the operation lever while firmly grasping the insertion section. US 6,981,945 discloses a handgrip for a colonoscope shaft capable of measuring and presenting to the operator radial and longitudinal forces applied by the operator during the manipulation of the colonoscope. The handgrip includes an internal sleeve positioned over the shaft of the colonoscope so that it can be released and repositioned by depressing a release button. An external sleeve covers the internal sleeve and provides a convenient grip for the operator. The operator operates engaging means provided to the external sleeve of the handgrip to apply forces to the shaft of the colonoscope. The engaging means are equipped with sensors adapted to sense the forces applied by the operator. These measurements are transmitted to an electronic unit for processing and display. The document US 6,981,945 discloses a device according to the preamble of claim 1.

It is an object of the present invention is to provide a user-friendly endoscopic operation assisting device capable of easily performing opening or closing operation of the handle and advancing or retreating operation of an endoscope, and also operation of a switch provided to the handle while grasping an insertion section of the endoscope through a grasping device.

This problem is solved by an endoscopic operation assisting device as defined in claim 1.

### SUMMARY OF THE INVENTION

An endoscopic operation assisting device according to the present invention is provided with a first handle member, a second handle member, and an operation switch. The first and the second handle members are openable and closable. The first handle member includes, in an opening and closing surface portion thereof, a groove forming an insertion section holding hole for holding an insertion section of an endoscope. Further, the first handle member includes, on a surface portion thereof contraposed to the opening and closing surface portion, a step portion including a flat surface which is provided at a distal end side disposed toward the direction of a distal end portion of the insertion section and which is lower than a proximal end side. Meanwhile, the second handle member includes, in an opening and closing surface portion thereof, a groove forming the insertion section holding hole. The operation switch is juxtaposed to one side surface of the flat surface included in the first handle member.

The above and other objects, features and advantages of the invention will become more clearly understood from the following description referring to the accompanying drawings.

### BRIEF DESCRIPTION OF THE DRAWINGS

Fig. 1 is a diagram illustrating a configuration of an endoscope system including an endoscopic operation assisting device;
Fig. 2 is a perspective view for explaining a state in which a surgeon grasps the endoscopic operation assisting device and operates an insertion section disposed in an insertion section holding hole;
Fig. 3 is a side view for explaining the endoscopic operation assisting device in the state of Fig. 2;
Fig. 4 is a diagram for explaining a first handle member and a second handle member and a hinge forming the endoscopic operation assisting device;
Fig. 5 is a front view for explaining the endoscopic operation assisting device brought into an open state by the biasing force of springs provided to hinges;
Fig. 6 is a perspective view for explaining the endoscopic operation assisting device in the open state;
Fig. 7 is a diagram illustrating the endoscopic operation assisting device in a closed state, as viewed from a distal end side;
Fig. 8 is a diagram illustrating the endoscopic operation assisting device in the closed state, as viewed from a proximal end side;
Fig. 9 is a cross-sectional view along the IX-IX line of Fig. 7;
Fig. 10 is a diagram for explaining the insertion section disposed in a sliding groove and a state in which a flat surface of an elastic member is in contact with the insertion section disposed in the sliding groove;
Fig. 11 is a diagram for explaining an indicator;
Fig. 12 is an exploded perspective view for explaining a configuration of an endoscopic operation assisting device, in which a connecting portion is formed by a sliding member;
Fig. 13 is a perspective view for explaining the endoscopic operation assisting device formed by the members of Fig. 12, as in an open state;
Fig. 14 is a diagram illustrating the endoscopic operation assisting device of Fig. 13, as viewed from a proximal end side; and
Fig. 15 is a diagram illustrating the endoscopic operation assisting device in the closed state, as viewed from the proximal end side.

### DETAILED DESCRIPTION OF PREFERRED EMBODIMENTS

Embodiments of the present invention will be described below with reference to the drawings.

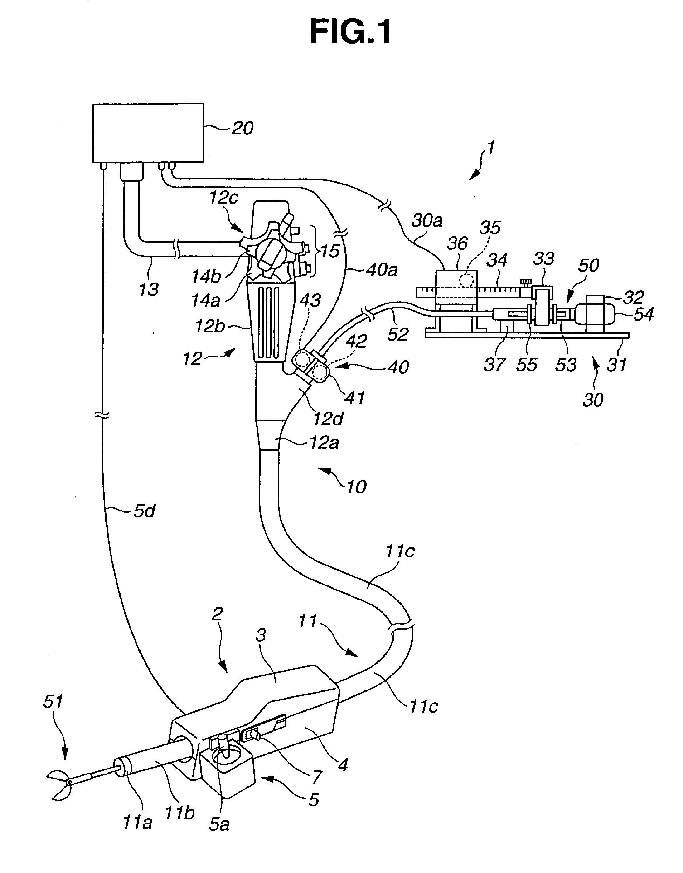

An embodiment of the present invention will be described with reference to Figs. 1 to 11.

With reference to Fig. 1, an endoscope system including an endoscopic operation assisting device will be described.

As illustrated in Fig. 1, an endoscope system 1 according to the present embodiment is configured to include an endoscopic operation assisting device (hereinafter described as the assisting device) 2, an endoscope 10, and external equipment of the endoscope 10, such as a control device 20, an electric treatment instrument opening and closing device 30, an electric treatment instrument advancing and retreating device 40, and so forth. The control device 20 according to the present embodiment serves as a light source device and a video processor, and is connected to not-illustrated display means, such as a monitor, which is external equipment for displaying an endoscopic image.

The endoscope 10 includes an insertion section 11, an operation section 12, and a universal cord 13. The operation section 12 is provided consecutively to the proximal end of the insertion section 11. The universal cord 13 extends from the operation section 12, and is connected to the control device 20.

The insertion section 11 includes a distal end portion 11a, a bending portion 11b, and a flexible tube portion 11c, which are consecutively provided in the above order from the distal end, and is configured as a tube member having flexibility. The operation section 12 is configured to include a bend preventing portion 12a provided consecutively to the proximal end of the insertion section 11, a grasping portion 12b including a treatment instrument insertion portion 12d, and a main operation portion 12c. The main operation portion 12c is provided with bending knobs 14a and 14b, switches for operating the air supply, the water supply, and the suction, and a plurality of switches 15 for sending a variety of instructions to image pickup means provided to the distal end portion 11a or to the control device 20.

The endoscope 10 includes a not-illustrated treatment instrument channel extending from the treatment instrument insertion portion 12d to an opening of the distal end portion 11 a of the insertion section 11.

The electric treatment instrument opening and closing device 30 is electrically connected to the control device 20 by an electrical cable 30a. The electric treatment instrument opening and closing device 30 is disposed with a handle portion 53 of a treatment instrument 50 which includes a medical instrument, such as a biopsy forceps, for example. The electric treatment instrument opening and closing device 30 is used in an opening or closing operation in which an opening operation or a closing operation of a treatment portion 51 is performed.

The electric treatment instrument advancing and retreating device 40 is electrically connected to the control device 20 by an electrical cable 40a. The electric treatment instrument advancing and retreating device 40 is used in an advancing or retreating operation in which a sheath 52 of the treatment instrument 50 is inserted in or withdrawn from the treatment instrument channel, and the device is disposed to the treatment instrument insertion portion 12d of the endoscope 10, for example.

The treatment instrument 50 according to the present embodiment includes the treatment portion 51 at the distal end of the sheath 52. The handle portion 53 is provided with a finger hook ring 54, and a slider 55 which can advance and retreat with respect to the handle portion 53. Through the sheath 52 of the treatment instrument 50, a not-illustrated operation wire is inserted which has an end connected to the treatment portion 51 and the other end connected to the slider 55. The treatment portion 51 performs opening and closing operations along with the advance and retreat of the operation wire.

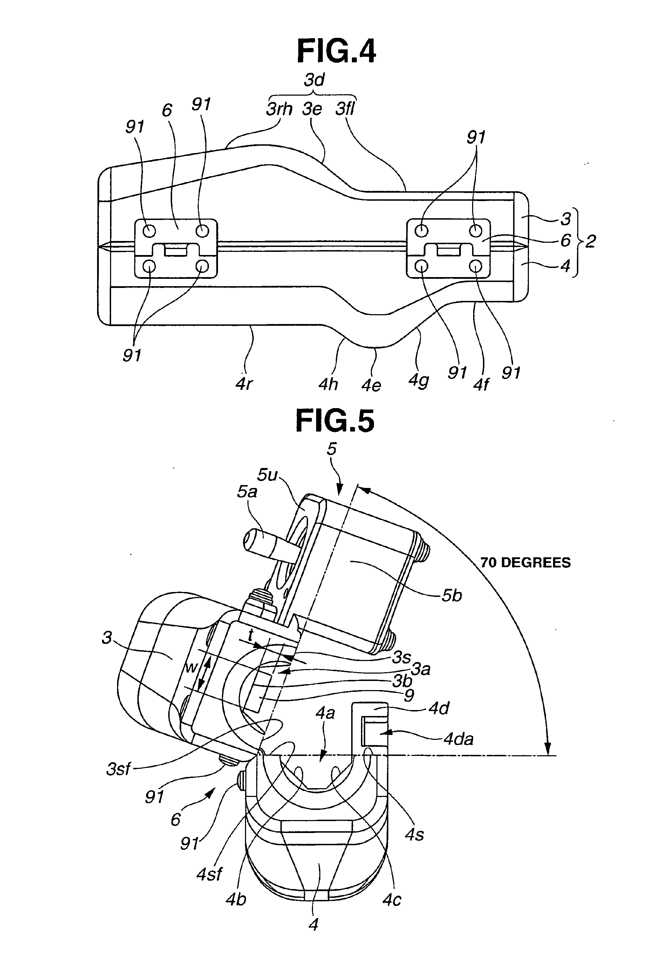

The assisting device 2 can be attachably and detachably disposed to the insertion section 11 of the endoscope 10, and particularly to the flexible tube portion 11c. A first handle member 3 and a second handle member 4 are configured to be openable and closable by hinges (see a reference numeral 6 in Fig. 4). A side surface of the first handle member 3 is attached with an operation switch (hereinafter abbreviated as the switch) 5 including an operation lever 5a for outputting an instruction signal for instructing the electric treatment instrument opening and closing device 30 and the electric treatment instrument advancing and retreating device 40 on the operation. The switch 5 is electrically connected to the control device 20 by a signal cable 5d.

The first handle member 3 is provided with a later-described fixing lever 7, which is used to fix the assisting device 2 to the insertion section 11.

The electric treatment instrument opening and closing device 30 is configured to include a base member 31, a ring retaining portion 32, a slider retaining portion 33, a rack 34, a motor 35, a holding box 36, and a mounting portion 37.

The ring retaining portion 32 is provided to project from the base member 31. The ring retaining portion 32 is inserted through a not-illustrated hole of the finger hook ring 54 of the treatment portion 50. The slider retaining portion 33 nips the slider 55 provided to the treatment instrument 50. The slider retaining portion 33 is fixed to one end portion of the rack 34 to be attachable and detachable with respect thereto, for example.

The rack 34 is provided with not-illustrated linear teeth. The linear teeth mesh with a not-illustrated pinion gear fixed to a motor shaft of the motor 35. Therefore, as the motor 35 is rotated in the clockwise direction or the counterclockwise direction, the rack 34 moves to advance or retreat together with the slider retaining portion 33. As the slider retaining portion 33 is advanced or retreated, the slider held by the slider retaining portion 33 moves to advance or retreat along the axis of the handle portion 53.

The holding box 36 is fixed to the base member 31. The holding box 36 holds the motor 35 and the rack 34. The mounting portion 37 is attached to the base member 31 to be integrated therewith, for example. On the mounting portion 37, the handle portion 53 of the treatment instrument 50 is mounted. When the instruction signal is outputted to the control device 20 from the switch 5, the motor 35 is driven under the control of a not-illustrated CPU of the control device 20.

The electric treatment instrument advancing and retreating device 40 includes a box member 41. Inside the box member 41, two rollers 42 and 43 are rotatably provided. Each of the two rollers 42 and 43 is formed by an elastic member, and the sheath 52 is disposed between the rollers 42 and 43. The sheath 52 is pressed and nipped by the rollers 42 and 43.

The first roller 42 is a drive roller, for example, and is rotated by a not-illustrated motor provided inside the box member 41. Meanwhile, the second roller 43 is a driven roller, and rotates along with the movement of the sheath 52, which is advanced or retreated by the rotational movement of the drive roller 42.

When the instruction signal is outputted from the switch 5 to the control device 20, the motor is driven under the control of the not-illustrated CPU of the control device 20. Further, the box member 41 is provided with a not-illustrated scope fixing portion for connecting the member to the treatment instrument insertion portion 12d, and a not-illustrated treatment instrument insertion portion in which the sheath 52 is inserted. The scope fixing portion is air-tightly connected to a channel opening of the treatment instrument insertion portion 12d, and a forceps plug formed by an elastic member is provided to a through hole of the treatment instrument insertion portion, through which the sheath 52 is inserted.

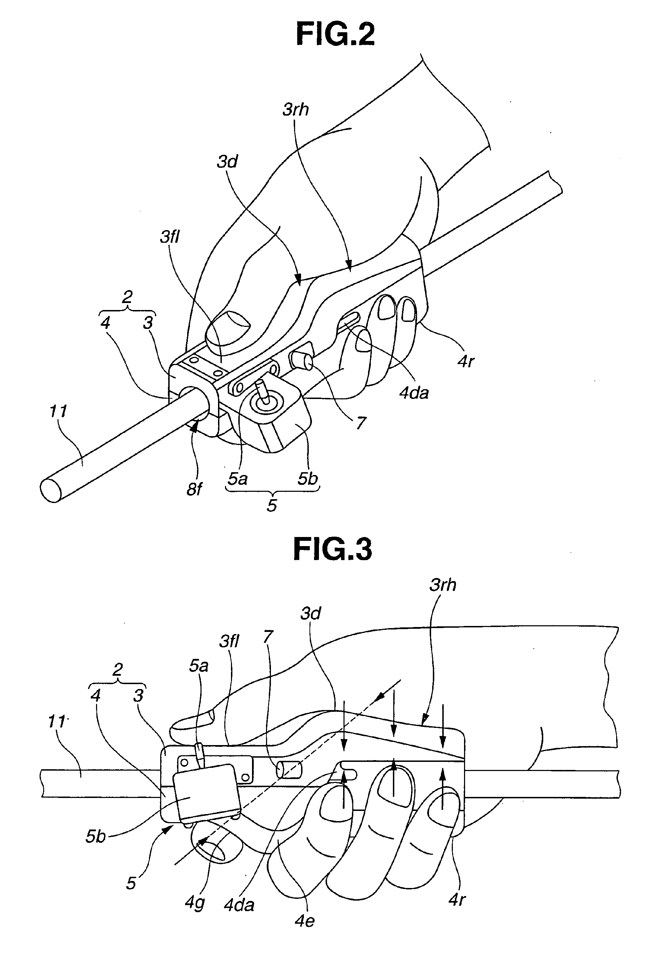

With reference to Figs. 1 to 9, the assisting device 2 will be described in detail. As illustrated in Figs. 2 and 3, the first handle member 3 and the second handle member 4 forming the assisting device 2 of the present embodiment illustrated in Fig. 1 are disposed to the flexible tube portion 11c, for example, which forms the insertion section 11 such that the first handle member 3 is on the upper side and the second handle member 4 is on the lower side in the figures during an operation, and the first handle member 3 and the second handle member 4 are grasped mainly by the right hand of a surgeon.

Herein, one end side of the assisting device 2 disposed toward the side of the distal end portion 11a of the insertion section 11 is described as the distal end side, while the other end side of the assisting device 2 disposed toward the proximal end side of the insertion section 11 opposed to the one end side is described as the proximal end side.

In a grasped state, the second handle member 4 of the assisting device 2 is grasped mainly by four singers of the surgeon excluding the thumb, as illustrated in the figures. Meanwhile, the thumb of the surgeon is placed on the first handle member 3 in the grasped state, as illustrated in the figures.

As illustrated in Fig. 4, the first handle member 3 and the second handle member 4 are connected by, for example, two rotatably configured hinges 6, which form a connecting portion. Specifically, the hinges 6 are fixed by screws 91 to a side surface of the first handle member 3, which forms the other side surface, and to a side surface of the second handle member 4, to thereby rotatably connect the first handle member 3 and the second handle member 4.

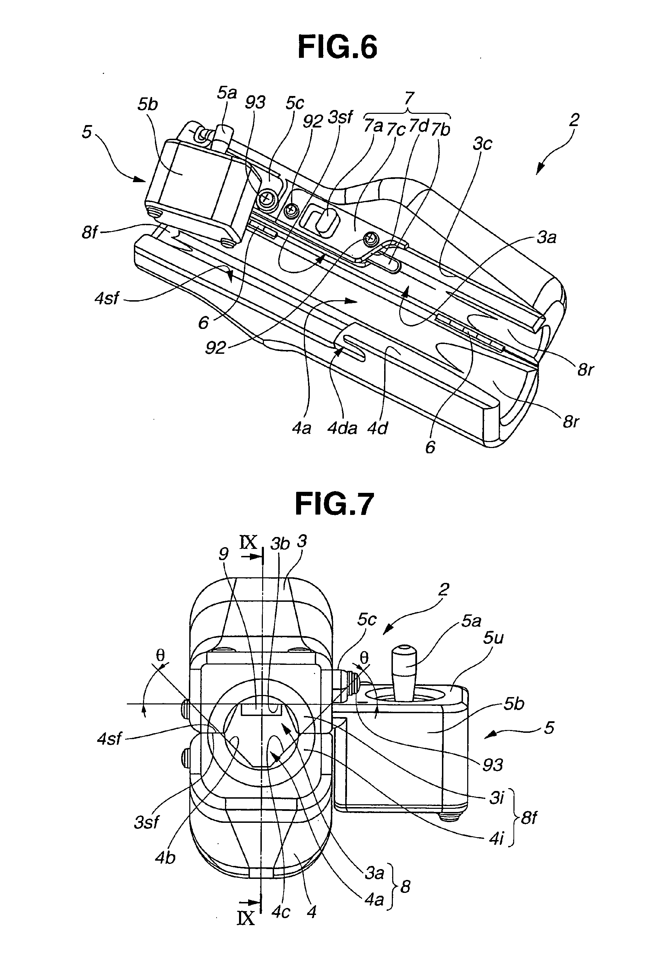

In the present embodiment, each of the hinges 6 is a hinge provided with a spring. Due to the biasing force of the spring, the first handle member 3 and the second handle member 4 are brought into an open state as illustrated in Figs. 5 and 6. Therefore, in the state in which the surgeon grasps the second handle member 4 with a plurality of fingers excluding the thumb, as illustrated in Fig. 2, for example, if the thumb of the surgeon is released from the first handle member 3, the first handle member 3 is moved to an open state position by the biasing force of the spring provided to the hinge 6. That is, in the present embodiment, the second handle member 4 forms a grasping member, while the first handle member 3 forms an opening and closing member.

Further, from the open state illustrated in Fig. 5, the first handle member 3 and the second handle member 4 are made approach each other against the biasing force of the spring provided to the hinge 6. Then, a distal-end-side opening and closing surface 3sf forming a distal end side of an opening and closing surface portion of the first handle member 3 is made in contact with a distal-end-side opening and closing surface 4sf forming a distal end side of an opening and closing surface portion of the second handle member 4. Thereby, the first handle member 3 and the second handle member 4 are brought into a closed state illustrated in Figs. 7, 8, and 9. In the closed state, the assisting device 2 is formed with an insertion section holding hole 8 for holding the flexible tube portion 11c.

In the open state illustrated in Fig. 5, a maximum open angle formed across the hinges 6 by the distal-end-side opening and closing surface 3sf of the first handle member 3 and the distal-end-side opening and closing surface 4sf of the second handle member 4 is seventy degrees. With the maximum open angle set at seventy degrees, when the thumb is placed on the first handle member 3 in the state in which the surgeon grasps the second handle member 4 with the four fingers excluding the thumb, the first handle member 3 is smoothly moved in a closing direction. In other words, when the thumb is placed on the first handle member 3, the first handle member 3 is prevented from being moved in the opposite direction to the closing direction.

In the present embodiment, the maximum open angle is set at seventy degrees. However, as long as the maximum open angle is set to be equal to or less than ninety degrees, the first handle member 3 can be prevented from moving in a direction in which the maximum open angle of the first handle member 3 exceeds the set angle when the thumb is placed on the first handle member 3 as described above.

As illustrated in Fig. 5 to 8, an opening and closing surface portion 3s of the first handle member 3 is formed with an insertion section pressing groove (hereinafter described as the pressing groove) 3a along the longitudinal axis of the first handle member 3. Meanwhile, an opening and closing surface portion 4s of the second handle member 4 is formed with an insertion section sliding disposition groove (hereinafter described as the sliding groove) 4a along the longitudinal axis of the second handle member 4.

The pressing groove 3a includes a flat surface 3b provided with an elastic member 9 forming a first contact surface which comes in contact with and presses an outer circumferential surface of the flexible tube portion 11c. The elastic member 9 includes a flat surface 9a which comes in contact with the outer circumferential surface of the flexible tube portion 11c, and is integrally attached to the flat surface 3b of the pressing groove 3a by adhesion, for example.

The flat surface 3b of the elastic member 9 has greater elastic force than flat surfaces 4b and 4c of the second handle member 4. Therefore, the holding force obtained by making the flat surface 3b of the elastic member 9 in contact with the flexible tube portion 11c is greater than the holding force obtained by making the flat surfaces 4b and 4c in contact with the flexible tube portion 11c. The width dimension w and the thickness dimension t of the elastic member 9 are set in consideration of the outer diameter dimension of the flexible tube portion 11c disposed in the insertion section holding hole 8. The length dimension of the member is set in consideration of later-described release portions 8f and 8r.

Meanwhile, the sliding groove 4a includes the flat surface 4b forming a second contact surface which comes in contact with the outer circumferential surface of the flexible tube portion 11c, and the flat surface 4c forming a third contact surface. Each of the flat surfaces 4b and 4c serves as a pressing surface which presses and holds the flexible tube portion 11c and an insertion section deposition surface on which the flexible tube portion 11c is disposed.

As illustrated in Fig. 7, in the closed state in which the distal-end-side opening and closing surface 3sf of the first handle member 3 and the distal-end-side opening and closing surface 4sf of the second handle member 4 are in contact with each other, the tilt angle of the first flat surface 4b with respect to the flat surface 3b is configured to be an angle θ, and the tilt angle of the second flat surface 4c with respect to the flat surface 3b is also configured to be the angle θ.

Further, in the closed state in which the opening and closing surface portion 3s of the first handle member 3 and the opening and closing surface portion 4s of the second handle member 4 are in contact with each other, the pressing groove 3a and the sliding groove 4a form, in the assisting device 2, the insertion section holding hole 8 elongated in the longitudinal direction in which the flexible tube portion 11c is disposed, as illustrated in Figs. 7 to 9.

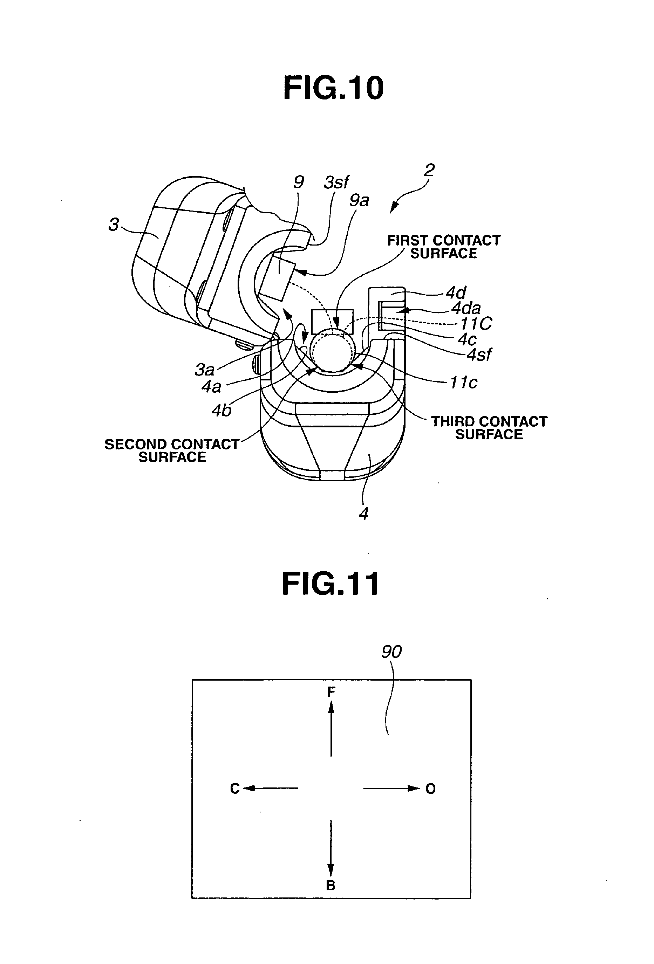

In the closed state, if the flexible tube portion 11c indicated by a solid line or a flexible tube portion 11C indicated by a broken line is disposed in the sliding groove 4a, as well as the flat surfaces 4b and 4c forming the sliding groove 4a, the flat surface 9a of the elastic member 9 comes in contact with the outer circumferential surface of the flexible tube portion 11c or 11C, as illustrated in Fig. 10. Thereby, the flexible tube portion 11c or 11C is so-called three-point supported by three surfaces provided to the insertion section holding hole 8, i.e., the first contact surface in contact with the flat surface 9a, the second contact surface in contact with the flat surface 4b, and the third contact surface in contact with the flat surface 4c.

That is, the insertion section holding hole 8 according to the present embodiment is configured to hold the insertion section, with the three surfaces of the flat surface 9a and the flat surfaces 4b and 4c made in contact with the outer circumferential surface of the insertion section 11. If the thickness dimension t of the elastic member 9 or the tilt angle of the flat surfaces 4b and 4c with respect to the flat surface 3b is suitably set, insertion sections of different outer diameter dimensions can be held in a balanced manner.

The insertion section holding hole 8 is provided with a distal-end-side release portion 8f and a proximal-end-side release portion 8r. Thus, in the state in which the flexible tube portion 11c is held by the assisting device 2, such a problem as damage caused by the biting of the assisting device 2 into the flexible tube portion 11c is prevented.

While preventing the biting, the distal-end-side release portion 8f is configured to prevent the positional displacement of the flexible tube portion 11c extending from the distal-end-side release portion 8f with respect to the insertion section holding hole 8 of the assisting device 2. On the other hand, while preventing the biting, the proximal-end-side release portion 8r is configured to prevent deterioration of the bending performance of the flexible tube portion 11c having flexibility and disposed behind the proximal-end-side release portion 8r.

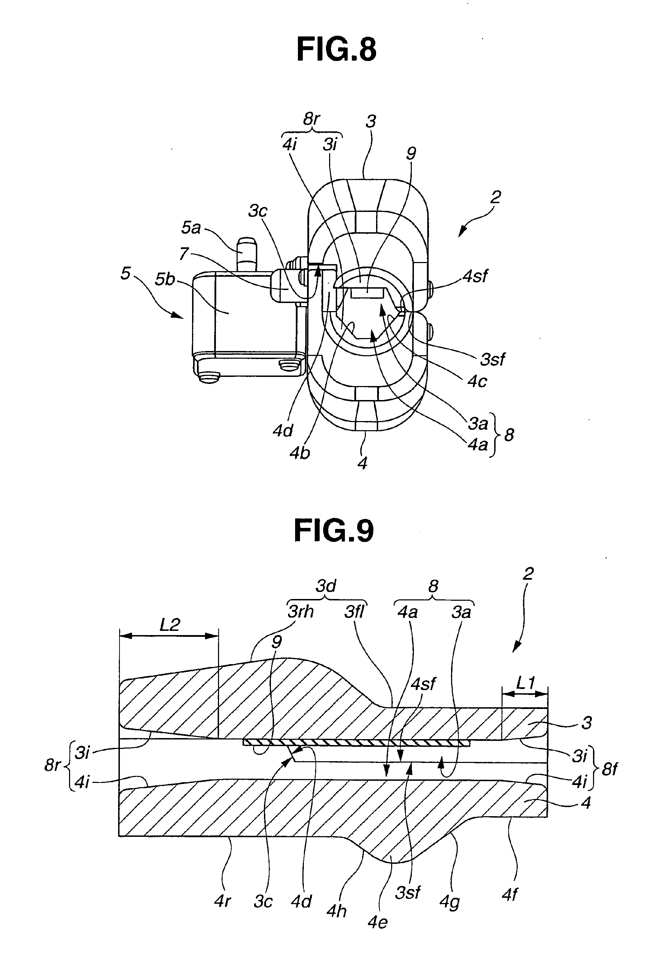

Specifically, as illustrated in Fig. 9, the distal-end-side release portion 8f and the proximal-end-side release portion 8r are formed by tilted surfaces 3i formed in the first handle member 3 and tilted surfaces 4i formed in the second handle member 4. In the tilted surfaces 3i and 4i forming the distal-end-side release portion 8f and the proximal-end-side release portion 8r, the tilt angles of the surfaces are set to be the same, for example, while the lengths of the surfaces in the longitudinal direction are changed. A length L1 of the distal-end-side release portion 8f is set to be shorter than a length L2 of the proximal-end-side release portion 8r to prevent the oscillation of the flexible tube portion 11c extending from the distal-end-side release portion 8f.

As illustrated in Figs. 5,6, and 9, the opening and closing surface portion 4s of the second handle member 4 is provided with a convex portion 4d projecting from the distal-end-side opening and closing surface 4sf. The convex portion 4d is formed to project from one of the side surfaces across the sliding groove 4a at a proximal end side of the opening and closing surface portion 4s. Meanwhile, the opening and closing surface portion 3s of the first handle member 3 is formed with a concave portion 3c in which the convex portion 4d is disposed. The concave portion 3c is formed to be recessed from one of the side surfaces across the pressing groove 3a at a proximal end side from the distal-end-side opening and closing surface 3sf.

With the convex portion 4d provided at a predetermined position at the proximal end side of the second handle member 4, the flexible tube portion 11c disposed on the flat surfaces 4b and 4c of the sliding groove 4a can be prevented from dropping from the sliding groove 4a even in the open state in which the distal-end-side opening and closing surface 3sf of the first handle member 3 and the distal-end-side opening and closing surface 4sf of the second handle member 4 are opened to the maximum extent, as illustrated in Fig. 10.

As illustrated in Figs. 1 to 4, the first handle member 3 includes a step portion 3d on an upper surface in the figures forming a surface portion contraposed to the opening and closing surface portion 3s. The step portion 3d is configured to include a distal end surface 3fl which is at the distal end side and forms a low surface of the step, and a proximal end surface 3rh which is at the proximal end side and forms a high surface of the step.

The distal end surface 3fl is a flat surface parallel to the distal-end-side opening and closing surface 3sf. When the surgeon operates the insertion section 11 while grasping the assisting device 2, the ball of the thumb of the surgeon is placed on the distal end surface 3fl. Meanwhile, the proximal end surface 3rh is a tilted surface tilted with respect to the distal end surface 3fl, and is configured such that the height thereof is gradually reduced toward the proximal end. When the surgeon operates the insertion section 11 while grasping the assisting device 2, a portion near the base of the thumb of the surgeon and a proximal palm portion are placed on the proximal end surface 3rh.

A step portion of the distal end surface 3fl and the proximal end surface 3rh is formed as a tilted surface in consideration of the graspability and the operability for the surgeon. A ridge portion of the distal end surface 3fl and the proximal end surface 3rh is formed as a curved surface in consideration of the graspability and the operability for the surgeon.

Meanwhile, the second handle member 4 includes a bulged portion 4e on a lower surface in the figures forming the other surface portion contraposed to the opening and closing surface portion 4s. The bulged portion 4e is provided in a mid portion on the lower surface of the second handle member 4. Therefore, the lower surface of the second handle member 4 is divided across the bulged portion 4e into a distal end surface 4f which is an end surface located at the distal end side and a proximal end surface 4r which is the other end surface located at the proximal end side.

A portion extending from the bulged portion 4e to the distal end surface 4f is formed as a bulged tilted surface 4g in consideration of the tilt of the proximal end surface 3rh of the first handle member 3. When the surgeon operates the insertion section 11 while grasping the assisting device 2, the forefinger of the surgeon is placed on the bulged tilted surface 4g.

A portion extending from the bulged portion 4e to the proximal end surface 4r is formed as a tilted surface 4h in consideration of the graspability and the operability for the surgeon. When the surgeon operates the insertion section 11 while grasping the assisting device 2, the middle finger of the surgeon is placed on the tilted surface 4h and the proximal end surface 4r, and the ring finger and the little finger are placed at the side of the proximal end surface 4r. A ridge of the distal end surface 4f, the bulged portion 4e, and the proximal end surface 4r of the second handle member 4 is formed as a curved surface in consideration of the graspability and the operability for the surgeon.

The fixing lever 7 is a lock mechanism portion for maintaining the closed state when the first handle member 3 and the second handle member 4 are brought into the closed state, and is configured as an elongated sliding member in the present embodiment. In the fixing lever 7, a knob 7a formed as a projection is provided at the distal end side, and a latch portion 7b disposed in a later-described latch concave portion 4da is provided at the proximal end side, as illustrated in Fig. 6. The fixing lever 7 is configured to be slidably disposed at a predetermined position on the first handle member 3, with an attachment plate 7c fixed by screws 92, for example, to a side surface of the first handle member 3.

The sliding axis of the fixing lever 7 and the longitudinal axis of the first handle member 3 are substantially parallel to each other. In other words, the sliding direction of the fixing lever 7 and the axial direction of the flexible tube portion 11c disposed in the insertion section holding hole 8 correspond to each other. Therefore, as the knob 7a is moved from an open position illustrated in Figs. 2 and 3 to a fixing position illustrated in Fig. 6, the latch portion 7b of the fixing lever 7 is projected from an attachment plate end surface 7d.

According to the above configuration, it is possible to prevent such a problem as damage on the surface of the flexible tube portion 11c caused as the flexible tube portion 11c is caught between the latch portion 7b and the second handle member 4 when the latch portion 7b of the fixing lever 7 is projected.

As illustrated in Figs. 5 and 6, a distal end surface side of the convex portion 4d provided to the second handle member 4 is formed with the latch concave portion 4da forming the lock mechanism portion, in which the latch portion 7b projecting from the attachment plate end surface 7d is disposed. As illustrated in Figs. 7 and 8, in the closed state in which the first handle member 3 and the second handle member 4 are closed, if the fixing lever 7 is moved from the open position illustrated in Figs. 2 and 3 to the fixing position illustrated in Fig. 6, the latch portion 7b of the fixing lever 7 is projected from the attachment plate end surface 7d, and thereafter is disposed in the latch concave portion 4da. Thereby, the first handle member 3 and the second handle member 4 in the closed state are maintained in the closed state, without being brought into the open state by the biasing force of the springs provided to the hinges 6.

The switch 5 includes the operation lever 5a which vertically projects from an upper surface 5u in the figures forming a surface of a case 5b. The case 5b is juxtaposed to a side surface of the first handle member 3 with the interposition of a fixing plate 5c. The fixing plate 5c is screwed into the first handle member 3 by screws 93, for example. In consideration of the operability of the operation lever 5a, the switch 5 juxtaposed to the first handle member 3 is tilted such that a proximal end side is higher than a distal end side in the upper surface 5u. Further, the height of an upper surface forming a distal end surface of the operation lever 5a is set to be substantially the same as the height of the distal end surface 3fl.

According to the switch 5 of the present embodiment, the opening or closing operation of the treatment portion 51 of the treatment instrument 50 or the advancing or retreating operation of the sheath 52 can be performed as the operation lever 5a is tilted in a predetermined direction by the surgeon, for example.

The switch 5, which is juxtaposed to the first handle member 3 with the fixing plate 5c screwed into the first handle member 3 by the screws 93, may be integrally fixed to the first handle member 3 by adhesion or the like, for example. Further, the distal end surface 3fl of the first handle member 3 is provided with an indicator 90 illustrated in Fig. 11 for instructing the directions of tilting operations, for example.

Specifically, in the present embodiment, when the surgeon tilts the operation lever 5a in the direction of F of the indicator 90, for example, the sheath 52 of the treatment instrument 50 can be advanced. Conversely, when the surgeon tilts the operation lever 5a in the direction of B of the indicator 90, the sheath 52 of the treatment instrument 50 can be retreated.

Meanwhile, when the surgeon tilts the operation lever 5a in the direction of O of the indicator 90, the treatment portion 51 of the treatment instrument 50 can be opened. Conversely, when the surgeon tilts the operation lever 5a in the direction of C of the indicator 90, the treatment portion 51 of the treatment instrument 50 can be closed.

That is, when the operation lever 5a is tilted in the direction of F or the direction of B of the indicator 90, the switch 5 output.s an instruction signal to the control device 20 via the signal cable 5d. Then, upon receipt of the instruction signal, the control device 20 supplies electric power to the electric treatment instrument advancing and retreating device 40 via the electrical cable 40a, and rotates the motor provided in the electric treatment instrument advancing and retreating device 40 in a predetermined direction. Then, the drive roller 42 is rotated along with the rotational movement of the motor. Thereby, the sheath 52 of the treatment instrument 50 nipped by the two rollers 42 and 43 is advanced or retreated inside the treatment instrument channel of the endoscope 10.

As a result, by tilting the operation lever 5a of the switch 5 in the direction of F or the direction of B of the indicator 90, the surgeon can perform the operation of drawing out the treatment portion 51 of the treatment instrument 50 into the body cavity through the insertion section 11 or the operation of withdrawing the treatment instrument 50 from the body cavity.

Meanwhile, when the operation lever 5a is tilted in the direction of O or the direction of C of the indicator 90, the switch 5 outputs an instruction signal to the control device 20 via the signal cable 5d. Then, upon receipt of the instruction signal, the control device 20 supplies electric power to the electric treatment instrument opening and closing device 30 via the electrical cable 30a, and rotates the motor 35 provided in the electric treatment instrument opening and closing device 30 in a predetermined direction. Then, the rack 34 is moved along with the rotational movement of the motor 35. Thereby, the slider 55 of the treatment instrument 50 held by the slider retaining portion 33 is advanced or retreated along the axis of the handle portion 53. Then, the operation wire of the treatment instrument 50 is pulled or slacked, and the treatment portion 51 of the treatment instrument 50 performs an opening operation or a closing operation.

As a result, by tilting the operation lever 5a of the switch 5 in the direction of O or the direction of C of the indicator 90, the surgeon can perform the operation of opening or closing the treatment portion 51 of the treatment instrument 50.

The operation of the assisting device 2 configured as described above will be described.

In the use of the endoscope system 1 illustrated in Fig. 1, a staff member prepares the assisting device 2. The assisting device 2 may be previously disposed at an arbitrary position of the flexible tube portion 11c of the insertion section 11 or may be disposed by the surgeon to the flexible tube portion 11c during an operation.

For example, in the case in which the assisting device 2 is previously disposed to the flexible tube portion 11c, the staff member places the first handle member 3 and the second handle member 4, which form the assisting device 2, in the open state, as illustrated in Fig. 6, and disposes the flexible tube portion 11c in the sliding groove 4a of the second handle member 4. Thereafter, the staff member places the first handle member 3 and the second handle member 4 in the closed state, and moves the knob 7a of the fixing lever 7 to the fixing position.

When inserting the insertion section 11 toward a lesion site, such as a polyp, located in the body cavity, the surgeon disposes the assisting device 2 to the flexible tube portion 11c. Alternatively, the surgeon uses the assisting device 2 previously fixed to the flexible tube portion 11c. When using the assisting device 2 fixed to the flexible tube portion 11c, the surgeon grasps the handle members 3 and 4, as illustrated in Figs. 2 and 3. Then, the surgeon moves the knob 7a of the fixing lever 7, which is disposed at the fixing position, to the open position.

The first handle member 3 of the assisting device 2 grasped by the surgeon can be constantly shifted to the open state by the basing force of the springs provided to the hinges 6. In other words, the surgeon grasps the first handle member 3 and the second handle member 4 against the basing force of the springs provided to the hinges 6.

Therefore, as the surgeon suitably changes the amount of force for grasping the first handle member 3 and the second handle member 4, the assisting device 2 shifts to a state in which the assisting device 2 smoothly moves with respect to the flexible tube portion 11 c or a state in which the assisting device 2 and the flexible tube portion 11c are firmly integrated with each other, for example.

Specifically, if the assisting device 2 is lightly grasped to be adjusted to a state in which the flat surface 9a of the elastic member 9 provided to the first handle member 3 is floated from the flexible tube portion 11c by the biasing force of the springs provided to the hinges 6, a state can be obtained in which the assisting device 2 smoothly moves with respect to the flexible tube portion 11c.

Meanwhile, if the assisting device 2 is firmly grasped to cause the three surfaces of the flat surface 9a of the elastic member 9 and the flat surfaces 4b and 4c forming the sliding groove 4a to come in contact with the outer circumferential surface of the flexible tube portion 11c, a state can be obtained in which the assisting device 2 and the flexible tube portion 11c are firmly integrated with each other. The above phenomenon is similar to the one in which the surgeon directly grasps the flexible tube portion 11c with his hand and changes the force for grasping the flexible tube portion 11c.

While viewing an endoscopic image, the surgeon suitably changes the amount of force for grasping the assisting device 2 to repeatedly perform the operation of inserting the insertion section 11 while holding the flexible tube portion 11c with the assisting device 2 and the operation of changing the position of the assisting device 2 with respect to the flexible tube portion 11c. Thereby, the insertion section 11 is inserted into the body cavity.

Then, when the lesion site is displayed in the endoscopic image, the surgeon moves only the thumb to suitably perform the tilting operations of the operation lever 5a of the switch 5 provided to the assisting device 2. Then, in accordance with the tilting operations of the operation lever 5a, the advancing and retreating operations of the sheath 52 and the opening and closing operations of the treatment portion 51 are performed to collect tissue, for example.

After the collection of the tissue, the insertion section 11 is withdrawn from the body cavity. In the process, the withdrawal of the insertion section 11 is performed with the use of the assisting device 2, for example.

To withdraw the insertion section 11 without the use of the assisting device 2, the assisting device 2 is detached from the insertion section 11, or the assisting device 2 is moved to a desired position at the proximal end side of the insertion section 11 and thereafter the knob 7a of the fixing lever 7 is moved from the open position to the fixing position to fix the assisting device 2 to the flexible tube portion 11c.

As described above, according to the assisting device 2 of the present embodiment, the twisting operation and the operations of inserting and withdrawing the insertion section 11 can be performed in a similar manner as in the case in which the surgeon directly grasps the insertion section 11 with his hand.

Further, the outer diameter of the assisting device 2 is larger than the outer diameter of the flexible tube portion 11c. Thus, the twisting operation of the insertion section 11 and so forth can be performed with a smaller amount of force than the amount of force used to directly grasp the flexible tube portion 11c with a hand.

Further, the first handle member 3 forming the assisting device 2 is provided with the step portion 3d, and the second handle member 4 is provided with the bulged portion 4e. Thus, when the surgeon grasps the assisting device 2, as illustrated in Fig. 2, the force applied by the hand and the fingers of the surgeons grasping the assisting device 2 as indicated by arrows of Fig. 3 is effectively transmitted to the first handle member 3 and the second handle member 4. Accordingly, the flexible tube portion 11c can be reliably grasped by the assisting device 2.

Further, with the assisting device 2 provided with the step portion 3d and the bulged portion 4e, when inserting the insertion section 11 into the body cavity while holding the flexible tube portion 11c with the assisting device 2, the hand and the fingers grasping the assisting device 2 are prevented from slipping by the step portion 3d or the bulged portion 4e. Therefore, the operations of inserting, withdrawing, and twisting the insertion section 11 can be reliably performed in a stable state.

Further, as the surgeon suitably moves the knob 7a of the fixing lever 7 provided to the assisting device 2 to the open position or the fixing position, it is possible to selectively obtain the state in which the assisting device 2 is movable with respect to the insertion section 11 and the state in which the assisting device 2 is fixed to the insertion section 11.

Further, in the configuration in which the three surfaces 9a, 4b, and 4c provided to the insertion section holding hole 8 are made in contact with the outer circumferential surface of the flexible tube portion 11c to hold the portion, the first handle member 3 forming the opening and closing member is provided with the elastic member 9 having large holding force. Thus, the assisting device 2 can be easily and smoothly moved with respect to the flexible tube portion 11c by adjusting the elastic member 9 of the first handle member 3 to the floating state.

Further, the proximal end side of the second handle member 4 is provided with the convex portion 4d. Thus, when the assisting device 2 is moved with respect to the insertion section 11 in the state in which the elastic member 9 of the first handle member 3 is floated, the assisting device 2 can be prevented from dropping from the insertion section 11. Furthermore, when the assisting device 2 is moved with respect to the insertion section 11, the assisting device 2 can be further smoothly moved with respect to the flexible tube portion 11c by making the convex portion 4d in contact with the flexible tube portion 11c.

Further, the switch 5 is juxtaposed to the first handle member 3. Thus, when the assisting device 2 is brought into the open state, the switch 5 is separated from the second handle member 4 together with the first handle member 3. Therefore, the insertion section 11 can be easily disposed in the sliding groove 4a provided in the second handle member 4.

Further, the switch 5 is juxtaposed to the first handle member 3, and the height of the distal end surface of the operation lever 5a is set to be the same as the height of the distal end surface 3fl of the first handle member 3. Thus, even in the state in which the assisting device 2 is firmly grasped, the operation of the operation lever 5a can be performed simply by slightly moving the thumb in a lateral direction without changing the grasping state of the assisting device 2. Therefore, the transition between the operation of grasping the assisting device 2 and the operation of operating the operation lever 5a is further smoothed.

Further, the height of the distal end surface of the operation lever 5a is the same as the height of the distal end surface 3f1 of the first handle member 3. Thus, it is possible to prevent a problem in which the thumb accidentally touches a side portion of the operation lever 5a during the operation of inserting the insertion section 11 with the use of the assisting device 2.

Further, the switch 5 juxtaposed to the first handle member 3 is tilted such that the proximal end side thereof is higher than the distal end side thereof. Thus, the operability of the operation lever 5a in the anteroposterior directions can be improved.

According to the above-described configurations, incorrect operation of the operation lever 5a is prevented.

Although the switch 5 is juxtaposed to the first handle member 3 in the present embodiment, the assisting device 2 may be configured such that the switch 5 is detached from the first handle member 3. If the switch 5 is detached from the first handle member 3, the switch 5 may be attached to the operation section 12, for example.

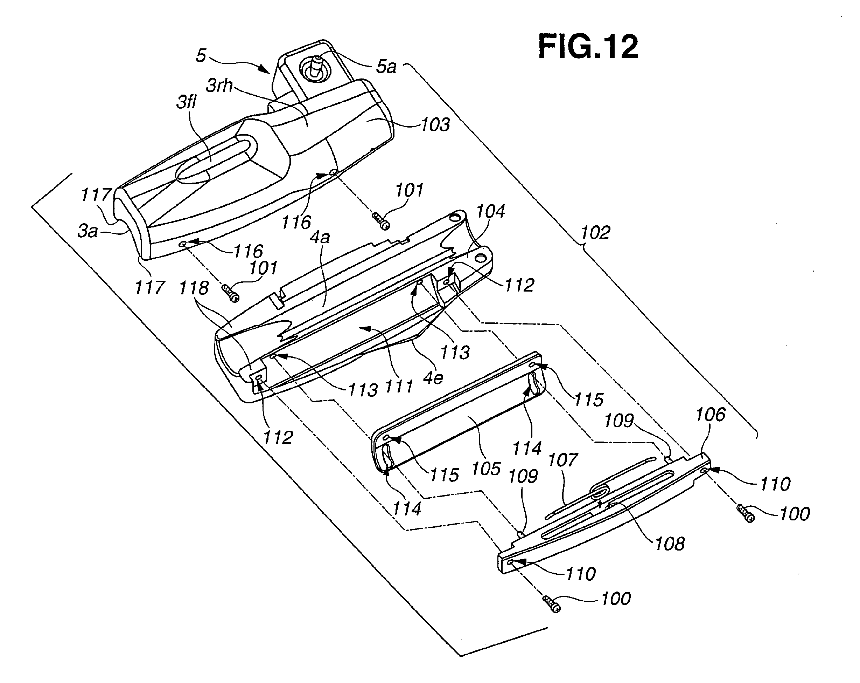

Further, in the present embodiment, the hinges 6 form connecting members. However, the connecting portion is not limited to the hinges. Thus, an assisting device 102 may be configured as illustrated in Figs. 12 to 15.

With reference to Figs. 12 to 15, description will be made of a configuration of the assisting device in which the connecting member is formed by a sliding member. The same members as the members of the above-described embodiment will be assigned with the same reference numerals, and description thereof will be omitted.

The assisting device 102 can be attachably and detachably disposed to the insertion section 11 of the endoscope 10, and particularly to the flexible tube portion 11c, and is configured to include a first handle member 103 and a second handle member 104, and a sliding plate 105 of a predetermined bent shape functioning as a sliding member and a rotational member, a sliding plate retaining plate (hereinafter abbreviated as the retaining plate) 106, and a biasing spring 107, which form a connecting portion.

The retaining plate 106 includes a spring space 108 in which the biasing spring 107 is disposed, a pair of projections 109, and counterbore holes 110 in which a pair of screws 100 are respectively disposed.

The second handle member 104 includes a sliding plate disposition portion 111 in which the sliding plate 105 is disposed, screw holes 112 into which the pair of screws 100 are respectively screwed, and holes 113 in which the pair of projections 109 are respectively disposed.

The sliding plate 105 includes oblong holes 114 through which the pair of projections 109 of the retaining plate 106 are respectively disposed, and screw holes 115 into which a pair of screws 101 are respectively screwed.

The first handle member 103 includes counterbore holes 116 in which the pair of screws 101 are respectively disposed.

To configure the assisting device 102, the biasing spring 107 is first disposed in the spring space 108 of the retaining plate 106, and the oblong holes 114 of the sliding plate 105 are disposed to the pair of projections 109 of the retaining plate 106. Thereafter, the sliding plate 105 is disposed in the sliding plate disposition portion 111 in a predetermined state, and the pair of projections 109 are disposed in the holes 113 of the second handle member 104. Then, the pair of screws 100 are screwed into the screw holes 112 through the respective counterbore holes 110.

Accordingly, the sliding plate 105 is disposed in a space formed by the second handle member 104 and the retaining plate 106 and including an opening on the side of the first handle member 103, such that the sliding plate 105 can slide by the length of the oblong holes 114.

Then, the sliding plate 105 and the first handle member 103 are fixed by the pair of screws 101. In the process, the sliding plate 105 is disposed to an inner surface of the first handle member 103 in a predetermined state, with the sliding plate 105 slid to project from the opening of the space by a predetermined amount. Thereafter, the pair of screws 101 are disposed in the respective counterbore holes 116 of the first handle member 103, and the screws 101 are screwed into the respective screw holes 115 provided in the sliding plate 105. Thereby, the first handle member 103 and the sliding plate 105 are integrally fixed to each other.

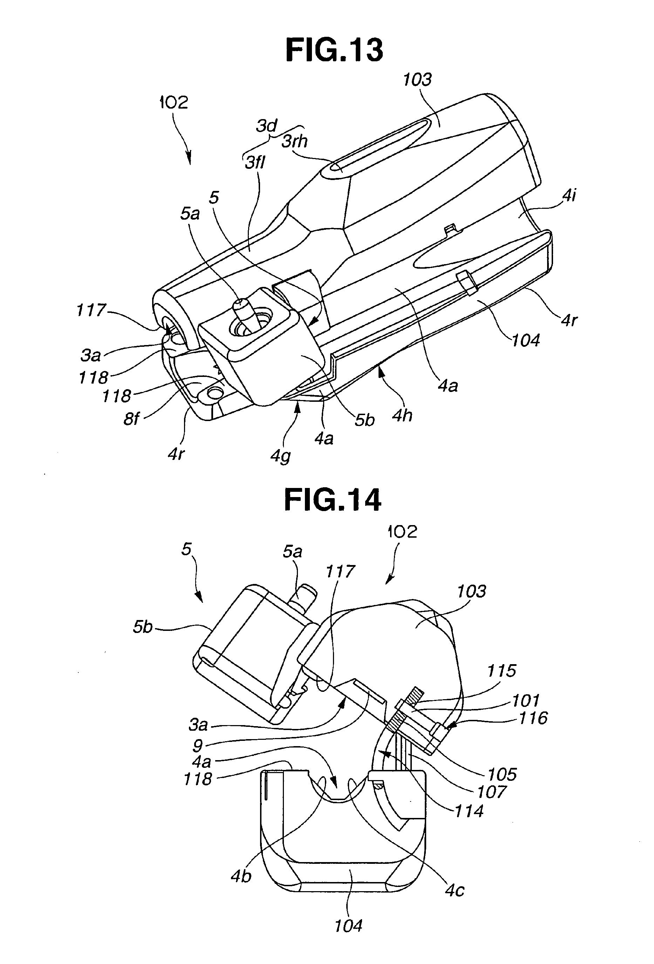

Accordingly, the assisting device 102 illustrated in Figs. 13 and 14 is formed. The first handle member 103 forming the assisting device 102 is moved, by the length of the oblong holes 114 formed in the sliding plate 105, by the biasing force of the biasing spring 107 disposed in the spring space 108 of the retaining plate 106. Thereby, the first handle member 103 and the second handle member 104 are brought into an open state in which the members are separated from each other.

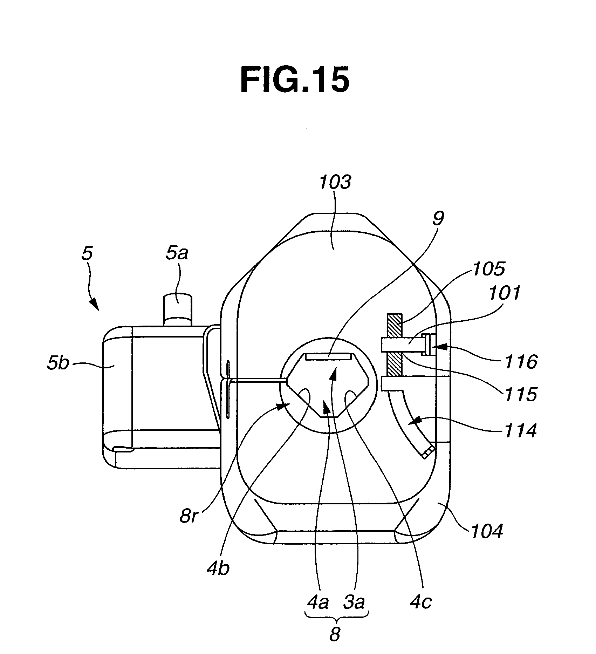

Further, also in the assisting device 102 according to the present embodiment, if the thumb is placed on the first handle member 103 in the state in which the surgeon grasps the second handle member 104 in a similar manner as in the above-described embodiment, for example, an opening and closing surface 117 of the first handle member 103 and an opening and closing surface 118 of the second handle member 104 are moved in the direction of coming in contact with each other. Then, along with the movement of the first handle member 103, the sliding plate 105 projecting from the space is stored in the space, as illustrated in Fig. 15, and the assisting device 102 is brought into a closed state.

If the thumb is released from the assisting device 102 in the closed state, for example, the sliding plate 105 is moved together with the first handle member 103 along the bent shape by the biasing force of the biasing spring 107. Thereby, the first handle member 103 and the second handle member 104 return to the open state in which the members are separated from each other.

As described above, the connecting portion is formed by the sliding plate 105 of the bent shape including the oblong holes 114 and the screw holes 115 and fixed to the first handle member 103 at one end thereof, the retaining plate 106 including the pair of projections 109 inserted through the oblong holes 114 to slidably attach the sliding plate 105 to the second handle member 104, and the biasing spring 107 disposed in the retaining plate 106 to bias the first handle member 103. Accordingly, the assisting device 102 can be configured as the openable and closable assisting device 102 in which the opening and closing surface 117 of the first handle member 103 and the opening and closing surface 118 of the second handle member 104 are separated from each other to be brought into the open state.

In the assisting device 102, the first handle member 103 and the second handle member 104 are not only rotationally moved but also linearly moved along the bent shape of the sliding plate 105 to shift from the closed state to the open state or from the open state to the closed state. Therefore, the insertion section 11 can be disposed in the sliding groove 4a, with the maximum open angle set to be equal to or less than forty-five degrees, for example.

Accordingly, when the thumb is placed on the first handle member 103, the first handle member 103 can be reliably prevented from moving in the direction in which the maximum open angle exceeds the set angle.

The other operations and advantages are similar to those of the above-described embodiment. Thus, the same members are assigned with the same reference numerals, and description thereof is omitted.

Having described the preferred embodiments of the invention referring to the accompanying drawings, it should be understood that the present invention is not limited to those precise embodiments.

## Claims

1. An endoscopic operation assisting device (2, 102) including a first handle member (3, 103) and a second handle member (4, 104), the first handle member (3, 103) and the second handle member (4, 104) being openable and closable, wherein the first handle member (3, 103) includes, in an opening and closing surface portion (3s) thereof, a groove (3a) forming an insertion section holding hole (8) for holding an insertion section (11) of an endoscope (10), and including, on a surface portion thereof contraposed to the opening and closing surface portion (3s), a step portion (3d) including a flat surface (3fl) which is provided at a distal end side of the first handle member (3, 103) and which is lower than a proximal end side surface (3rh), and
wherein the second handle member (4, 104) includes, in an opening and closing surface portion (4s) thereof, a groove (4a) forming the insertion section holding hole (8), **characterized in that** the endoscopic operation assisting device (2, 102) further comprises an operation switch (5) including a case (5b) which is juxtaposed to a side surface of the first handle member (3, 103), wherein the operation switch (5) is operable to output an instruction signal to a control device (20) for instructing operation of external equipment of an endoscope (10), and wherein the switch includes an operation lever (5a) which vertically projects from a surface of the switch.

2. The endoscopic operation assisting device according to Claim 1,
wherein the operation switch (5) is operable to output an instruction signal to a control device (20) for instructing operation of an electric treatment instrument opening and closing device (30) and for instructing operation of an electric treatment instrument advancing and retreating device (40).

3. The endoscopic operation assisting device according to Claim 1 or 2,
wherein the second handle member (4, 104) includes a bulged portion (4e) disposed on a surface portion contraposed to the opening and closing surface portion (4s) of the second handle member (4, 104) and disposed between a distal-end-side surface portion (4f) and a proximal-end-side surface portion (4r).

4. The endoscopic operation assisting device according to Claim 3,
wherein when the first handle member (3, 103) and the second handle member (4, 104) are brought into a closed state, the bulged portion (4e) is located at a more distal end side than the step portion (3d) of the first handle member (3, 103).

5. The endoscopic operation assisting device according to Claim 3,
wherein the bulged portion (4e) includes a tilted surface (4g) leading to the distal-end-side surface portion, and
a proximal-end-side surface (3rh) of the first handle member (3, 103) forms a tilted surface opposed to the tilted surface (4g).

6. The endoscopic operation assisting device according to Claim 1,
wherein the insertion section holding hole (8), which is formed by the groove (3a) included in the first handle member (3, 103) and the groove (4a) included in the second handle member (4, 104) when the first handle member (3, 103) and the second handle member (4, 104) are brought into a closed state, includes three contact surfaces (9a, 4b, 4c) adapted to contact an outer circumferential surface of an insertion section (11) of an endoscope (10) disposed in the insertion section holding hole (8).

7. The endoscopic operation assisting device according to Claim 6,
wherein the three contact surfaces comprise a first contact surface (9a) formed by an elastic member disposed in the groove (3a) of the first handle member (3, 103), and a second contact surface (4b) and a third contact surface (4c) formed by surfaces forming the groove (4a) of the second handle member (4, 104).

8. The endoscopic operation assisting device according to Claim 1,
wherein the second handle member (4, 104) includes a convex portion on one of the sides across the groove (4a) and at a proximal end side of the opening and closing surface portion (4s) of the second handle member (4, 104), and
wherein the first handle member (3, 103) includes, at a proximal end side of the opening and closing surface portion (3s) of the first handle member (3, 103), a concave portion in which the convex portion is disposed when the first handle (3, 103) member and the second handle member (4, 104) are brought into a closed state.

9. The endoscopic operation assisting device according to Claim 1, further comprising a lock mechanism portion for maintainings the first handle member (3, 103) and the second handle member (4, 104) in the closed state when the first handle member (3, 103) and the second handle member (4, 104) are brought into the closed state, the lock mechanism portion comprising:
a lever (7) provided to the first handle member (3, 103), being movable to advance and retreat in longitudinal directions, and including a knob (7a) and a latch portion (7b); and
a latch concave portion (4da) formed on the second handle member(4da), wherein the latch portion (7b) of the lever (7) is disposed in the latch concave portion (4da) when the first handle member (3, 103) and the second handle member (4, 104) are brought into the closed state.

10. The endoscopic operation assisting device according to Claim 9,
wherein the latch concave portion (4da) is provided to a convex portion of the second handle member (4, 104).

11. The endoscopic operation assisting device according to Claim 1,
wherein the height of a distal end surface of the operation lever (5a) provided to the operation switch (5) corresponds to the height of the flat surface (3fl).

12. The endoscopic operation assisting device according to Claim 1 or 11,
wherein a surface forming the operation switch is tilted with respect to the flat surface (3fl), and the tilt of the surface is higher at a proximal end side thereof than at a distal end side thereof.

13. The endoscopic operation assisting device according to any one of 1, 11 or 12
wherein the operation switch (5) is attachable and detachable with respect to the first handle member (3, 103).

14. The endoscopic operation assisting device according to Claim 1,
wherein the first handle member (3, 103) and the second handle member (4, 104) are connected by a connecting portion.

15. The endoscopic operation assisting device according to Claim 14,
wherein the connecting portion is a hinge (9) and shifts the first handle member (3, 103) and the second handle member (4, 104) from an open state to a closed state or from a closed state to an open state through a rotational movement thereof.

16. The endoscopic operation assisting device according to Claim 15,
wherein the hinge (9) is a hinge (9) provided with a spring.

17. The endoscopic operation assisting device according to Claim 14,
wherein the connecting portion includes:
a sliding plate (105) of a bent shape including oblong holes (114), and screw holes (110) for integrally fixing the sliding plate (105) to the first handle member (103);
a sliding plate retaining plate (106) including convex portions (109) disposed through the oblong holes (114) of the sliding plate (105) in holes formed in the second handle member (4, 104) to slidably attach the sliding plate (105) to the second handle member (4, 104); and
a biasing spring (107) provided to the sliding plate retaining plate (106) to bias the first handle member (3, 103), to which the sliding plate (105) is integrally fixed, and
wherein, when the first handle member (3, 103) and the second handle member (4, 104) are brought into a closed state against the biasing force of the biasing spring (107), the opening and closing surface of the first handle member (3, 103) and the opening and closing surface of the second handle member (4, 104) come in contact with each other, and when the first handle member (3, 103) and the second handle member (4, 104) are brought into an open state by the biasing force of the biasing spring (107), the opening and closing surface of the first handle member (3, 103) and the opening and closing surface of the second handle member (4, 104) are separated from each other to be brought into the open state.

## Patentansprüche

1. Endoskopbetätigungshilfsgerät (2, 102), das ein erstes Griffelement (3, 103) und ein zweites Griffelement (4, 104) umfasst, wobei das erste Griffelement (3, 103) und das zweite Griffelement (4, 104) geöffnet und geschlossen werden können, wobei das erste Griffelement (3, 103) in einem Öffnungs- und Schließoberflächenabschnitt (3s) eine Nut (3a) umfasst, die eine Einführabsch, nitthalteöffnung (8) zum Halten eines Einführabschnitts (11) eines Endoskops (10) bildet und auf einem Oberflächenabschnitt gegenüber dem Öffnungs- und Schließoberflächenabschnitt (3s), einen Stufenabschnitt (3d) umfasst, der eine flache Oberfläche (3fl) umfasst, die an einer distalen Endseite des ersten Griffelements (3, 103) vorgesehen ist und die niedriger als eine proximale Endseitenoberfiläche (3rh) ist, und
wobei das zweite Griffelement (4, 104) in einem Öffnungs- und Schließoberflächenabschnitt (4s) eine Nut (4a) umfasst, die die Einführabschnitthalteöffnung (8) bildet, **dadurch gekennzeichnet, dass** das Endoskopbetätigungshilfsgerät (2, 102) ferner einen Betätigungsschalter (5) umfasst, der ein Gehäuse (5b) umfasst, das neben einer Seitenoberfläche des ersten Griffelements (3, 103) angeordnet ist, wobei der Betätigungsschalter (5) dazu eingerichtet ist, ein Instruktionssignal zum Instruieren einer Betätigung eines externen Geräts eines Endoskops (10) an ein Steuergerät (20) auszugeben, und wobei der Schalter einen Betätigungshebel (5A) umfasst, der vertikal von einer Oberfläche des Schalters hervorsteht.

2. Endoskopbetätigungshilfsgerät gemäß Anspruch 1,
wobei der Betätigungsschalter (5) dazu eingerichtet ist, ein Instruktionssignal zum Instruieren einer Betätigung eines elektrischen Geräts (30) zum Öffnen und Schließen eines Behandlungsinstruments und zum Instruieren einer Betätigung eines elektrischen Geräts (40) zum Vorschieben und Zurückziehen eines Behandlungsinstruments an ein Steuergerät (20) auszugeben.

3. Endoskopbetätigungshilfsgerät gemäß Anspruch 1 oder 2,
wobei das zweite Griffelement (4, 104) einen gewölbten Abschnitt (4e) umfasst, der an einem Oberflächenabschnitt gegenüber dem Öffnungs- und Schließoberflächenabschnitt (4s) des zweiten Griffelements (4, 104) vorgesehen ist und der zwischen einem distalen Endseitenoberflächenabschnitt (4f) und einem proximalen Endseitenoberflächenabschnitt (4r) vorgesehen ist.

4. Endoskopbetätigungshilfsgerät gemäß Anspruch 3,
wobei, wenn das erste Griffelement (3, 103) und das zweite Griffelement (4, 104) in einen geschlossenen Zustand gebracht sind, der gewölbte Abschnitt (4e) weiter auf einer distalen Endseite gelegen ist als der Stufenabschnitt (3d) des ersten Griffelements (3, 103).

5. Endoskopbetätigungshilfsgerät gemäß Anspruch 3,
wobei der gewölbte Abschnitt (4e) eine geneigte Oberfläche (4g) umfasst, die zu dem distalen Endseitenoberflächenabschnitt führt, und
eine proximale Endseitenoberfläche (3rh) des ersten Griffelements (3, 103) eine geneigte Oberfläche bildet, die der geneigten Oberfläche (4g) gegenüberliegt.

6. Endoskopbetätigungshilfsgerät gemäß Anspruch 1,
wobei die Einführabschnitthalteöffnung (8), die durch die in dem ersten Griffelement (3, 103) vorgesehene Nut (3a) und die in dem zweiten Griffelement (4, 104) vorgesehene Nut (4a) gebildet wird, wenn das erste Griffelement (3, 103) und das zweite Griffelement (4, 104) in einen geschlossenen Zustand gebracht sind, drei Kontaktoberflächen (9a, 4b, 4c) umfasst, die dazu eingerichtet sind, eine äußere Umfangsoberfläche eines Einführabschnitts (11) eines Endoskops (10) zu berühren, der in der Einführabschnitthalteöffnung (8) angeordnet ist.

7. Endoskopbetätigungshilfsgerät gemäß Anspruch 6,
wobei die drei Kontaktoberflächen eine erste Kontaktoberfläche (9a), die durch ein in der Nut (3a) des ersten Griffelements (3, 103) angeordnetes elastisches Element gebildet wird, und eine zweiten Kontaktoberfläche (4b) und eine dritten Kontaktoberfläche (4c) umfassen, die durch die Nut (4a) des zweiten Griffelements (4, 104) bildende Oberflächen gebildet werden.

8. Endoskopbetätigungshilfsgerät gemäß Anspruch 1,
wobei das zweite Griffelement (4, 104) einen konvexen Abschnitt auf einer der Seiten über die Nut (4a) und an einer proximalen Endseite des Öffnungs- und Schließoberflächenabschnitts (4s) des zweiten Griffelements (4, 104) umfasst, und
wobei das erste Griffelement (3, 103) an einer proximalen Endseite des Öffnungs- und Schließoberflächenabschnitts (3s) des ersten Griffelement (3, 103) einen konkaven Abschnitt umfasst, in dem der konvexe Abschnitt angeordnet ist, wenn das erste Griffelement (3, 103) und das zweite Griffelement (4, 104) in einen geschlossenen Zustand gebracht sind.

9. Endoskopbetätigungshilfsgerät gemäß Anspruch 1, das ferner einen Verschlussmechanismusabschnitt zum Halten des ersten Griffelements (3, 103) und des zweiten Griffelements (4, 104) in dem geschlossenen Zustand umfasst, wenn das erste Griffelement (3, 103) und das zweite Griffelement (4, 104) in den geschlossenen Zustand gebracht sind, wobei der Verschlussmechanismusabschnitt umfasst:
einen Hebel (7), der an dem ersten Griffelement (3, 103) vorgesehen ist, in Längsrichtungen vorwärts und rückwärts bewegbar ist und einen Knopf (7a) und einen Riegelabschnitt (7b) umfasst; und
einen konkaven Riegelabschnitt (4da), der an dem zweiten Griffelement (4, 104) ausgebildet ist, wobei der Riegelabschnitt (7b) des Hebels (7) in dem konkaven Riegelabschnitt (4da) angeordnet ist, wenn das erste Griffelement (3, 103) und das zweite Griffelement (4, 104) in den geschlossenen Zustand gebracht sind.

10. Endoskopbetätigungshilfsgerät gemäß Anspruch 9,
wobei der konkave Riegelabschnitt (4da) an einem konvexen Abschnitt des zweiten Griffelements (4, 104) vorgesehen ist.

11. Endoskopbetätigungshilfsgerät gemäß Anspruch 1,
wobei die Höhe einer distalen Endoberfläche des Betätigungshebels (5a), der an dem Betätigungsschalter (5) vorgesehen ist, der Höhe der flachen Oberfläche (3fl) entspricht.

12. Endoskopbetätigungshilfsgerät gemäß Anspruch 1 oder 11,
wobei eine den Betätigungsschalter bildende Oberfläche bezüglich der flachen Oberfläche (3fl) geneigt ist und die Neigung der Oberfläche an ihrer proximalen Endseite größer ist als an ihrer distalen Endseite.

13. Endoskopbetätigungshilfsgerät gemäß einem der Ansprüche 1 oder 11,
wobei der Betätigungsschalter (5) bezüglich des ersten Griffelements (3, 103) befestigbar und lösbar ist.

14. Endoskopbetätigungshilfsgerät gemäß Anspruch 1,
wobei das erste Griffelement (3, 103) und das zweite Griffelement (4, 104) durch einen Verbindungsabschnitt verbunden sind.

15. Endoskopbetätigungshilfsgerät gemäß Anspruch 14,
wobei der Verbindungsabschnitt ein Scharnier (9) ist und das erste Griffelement (3, 103) und das zweite Griffelement (4, 104) durch eine Rotationsbewegung aus einem geöffneten Zustand in einen geschlossenen Zustand oder aus einem geschlossenen Zustand in einen geöffneten Zustand verschiebt.

16. Endoskopbetätigungshilfsgerät gemäß Anspruch 15,
wobei das Scharnier (9) ein Scharnier (9) ist, das mit einer Feder versehen ist.

17. Endoskopbetätigungshilfsgerät gemäß Anspruch 14,
wobei der Verbindungsabschnitt umfasst:
eine Gleitplatte (105) mit einer gebogenen Form, die Langlöcher (114) und Schraubenlöcher (110) zum integrierten Befestigen der Gleitplatte (105) an dem ersten Griffelement (103) umfasst;
eine Gleitplattenhalteplatte (106), die konvexe Abschnitte (109) umfasst, die durch die Langlöcher (114) der Gleitplatte (105) in in dem zweiten Griffelement (4, 104) ausgebildeten Öffnungen angeordnet sind, um die Gleitplatte (105) gleitend an dem zweiten Griffelement (4, 104) zu befestigen; und
eine Vorspannfeder (107), die an der Gleitplattenhalteplatte (106) vorgesehen ist, um das erste Griffelement (3, 103), an dem die Gleitplatte (105) integriert befestigt ist, vorzuspannen, und
wobei, wenn das erste Griffelement (3, 103) und das zweite Griffelement (4, 104) entgegen der Vorspannkraft der Vorspannfeder (107) in einen geschlossenen Zustand gebracht sind, die Öffnungs- und Schließoberfläche des ersten Griffelements (3, 103) und die Öffnungs- und Schließoberfläche des zweiten Griffelements (4, 104) miteinander in Kontakt geraten, und wenn das erste Griffelement (3, 103) und das zweite Griffelement (4, 104) durch die Vorspannkraft der Vorspannfeder (107) in einen geöffneten Zustand gebracht sind, die Öffnungs- und Schließoberfläche des ersten Griffelements (3, 103) und die Öffnungs- und Schließoberfläche des zweiten Griffelements (4, 104) voneinander getrennt sind, um in einen geöffneten Zustand gebracht zu werden.

## Revendications

1. Dispositif (2, 102) d'assistance pour une opération endoscopique comprenant un premier élément de poignée (3, 103) et un deuxième élément de poignée (4, 104), le premier élément de poignée (3, 103) et le deuxième élément de poignée (4, 104) pouvant être ouverts et fermés, dans lequel le premier élément de poignée (3, 103) comprend, dans une partie (3s) de surface d'ouverture et de fermeture de celui-ci, une rainure (3a) formant un trou (8) de maintien de section d'insertion destinée à maintenir une section d'insertion (11) d'un endoscope (10), et incluant, sur une partie de surface de celui-ci opposée à la partie (3s) de surface d'ouverture et de fermeture, une partie étagée (3d) incluant une surface plate (3fl) qui est prévue au niveau d'un côté d'extrémité distale du premier élément de poignée (3, 103) et qui est inférieure à une surface (3rh) du côté d'extrémité proximale, et
dans lequel le deuxième élément de poignée (4, 104) comprend, dans une partie (4s) de surface d'ouverture et de fermeture de celui-ci, une rainure (4a) formant le trou (8) de maintien de section d'insertion, **caractérisé en ce que** le dispositif (2, 102) d'assistance pour opération endoscopique comprend en outre un commutateur de fonctionnement (5) incluant un boîtier (5b) qui est juxtaposé sur une surface latérale du premier élément de poignée (3, 103), dans lequel le commutateur de fonctionnement (5) est utilisable pour délivrer en sortie un signal d'instruction vers un dispositif de commande (20) destiné à instruire une opération d'un équipement externe d'un endoscope (10), et dans lequel le commutateur comprend un levier de commande (5a) qui fait saillie verticalement depuis une surface du commutateur.

2. Dispositif d'assistance pour une opération endoscopique selon la revendication 1,
dans lequel le commutateur de fonctionnement (5) est utilisable pour délivrer en sortie un signal d'instruction vers un dispositif de commande (20) pour instruire une opération d'un dispositif (30) d'ouverture et de fermeture d'instrument de traitement électrique et pour instruire une opération d'un dispositif (40) d'avance et de recul d'instrument de traitement électrique.

3. Dispositif d'assistance pour une opération endoscopique selon la revendication 1 ou 2,
dans lequel le deuxième élément de poignée (4, 104) comprend une partie bombée (4e) disposée sur une partie de surface opposée à la partie (4s) de surface d'ouverture et de fermeture de deuxième élément de poignée (4, 104) et disposée entre une partie (4f) de surface du côté d'extrémité distale et une partie (4r) de surface du côté d'extrémité proximale.

4. Dispositif d'assistance pour une opération endoscopique selon la revendication 3,
dans lequel lorsque le premier élément de poignée (3, 103) et le deuxième élément de poignée (4, 104) sont amenés dans un état fermé, la partie bombée (4e) est placée plus près du côté d'extrémité distale que la partie étagée (3d) du premier élément de poignée (3, 103).

5. Dispositif d'assistance pour une opération endoscopique selon la revendication 3,
dans lequel la partie bombée (4e) comprend une surface inclinée (4g) menant à la partie de surface du côté d'extrémité distale, et
une surface (3rh) du côté d'extrémité proximale du premier élément de poignée (3, 103) forme une surface inclinée opposée à la surface inclinée (4g).

6. Dispositif d'assistance pour une opération endoscopique selon la revendication 1,
dans lequel le trou (8) de maintien de section d'insertion, qui est formé par la rainure (3a) contenue dans le premier élément de poignée (3, 103) et la rainure (4a) contenue dans le deuxième élément de poignée (4, 104) lorsque le premier élément de poignée (3, 103) et le deuxième élément de poignée (4, 104) sont amenés dans un état fermé, comprend trois surfaces de contact (9a, 4b, 4c) adaptées pour contacter une surface circonférentielle externe d'une section d'insertion (11) d'un endoscope (10) disposée dans le trou (8) de maintien de section d'insertion.

7. Dispositif d'assistance pour une opération endoscopique selon la revendication 6,
dans lequel les trois surfaces de contact comprennent une première surface de contact (9a) formée par un élément élastique disposé dans la rainure (3a) du premier élément de poignée (3, 103), et une deuxième surface de contact (4b) et une troisième surface de contact (4c) formées par les surfaces formant la rainure (4a) du deuxième élément de poignée (4, 104).

8. Dispositif d'assistance pour une opération endoscopique selon la revendication 1,
dans lequel le deuxième élément de poignée (4, 104) comprend une partie convexe sur l'un des côtés à travers la rainure (4a) et au niveau d'un côté d'extrémité proximale de la partie (4s) de surface d'ouverture et de fermeture du deuxième élément de poignée (4, 104), et
dans lequel le premier élément de poignée (3, 103) comprend, au niveau d'un côté d'extrémité proximale de la partie (3s) de surface d'ouverture et de fermeture du premier élément de poignée (3, 103), une partie concave dans laquelle la partie convexe est disposée lorsque le premier élément de poignée (3, 103) et le deuxième élément de poignée (4, 104) sont amenés dans un état fermé.

9. Dispositif d'assistance pour une opération endoscopique selon la revendication 1, comprenant en outre une partie de mécanisme de verrouillage destinée à maintenir le premier élément de poignée (3, 103) et le deuxième élément de poignée (4, 104) dans l'état fermé lorsque le premier élément de poignée (3, 103) et le deuxième élément de poignée (4, 104) sont amenés dans l'état fermé, la partie du mécanisme de verrouillage comprenant :
un levier (7) prévu sur le premier élément de poignée (3, 103), étant mobile pour avancer et reculer dans les directions longitudinales, et incluant un bouton (7a) et une partie de verrouillage (7b) ; et
une partie concave (4da) de verrouillage formée sur le deuxième élément de poignée (4, 104), dans laquelle la partie de verrouillage (7b) du levier (7) est disposée dans la partie concave (4da) de verrouillage lorsque le premier élément de poignée (3, 103) et le deuxième élément de poignée (4, 104) sont amenés dans l'état fermé.

10. Dispositif d'assistance pour une opération endoscopique selon la revendication 9,
dans lequel la partie concave (4da) de verrouillage est prévue sur une partie convexe du deuxième élément de poignée (4, 104).

11. Dispositif d'assistance pour une opération endoscopique selon la revendication 1,
dans lequel la hauteur d'une surface d'extrémité distale du levier de commande (5a) prévu sur le commutateur de fonctionnement (5) correspond à la hauteur de la surface plate (3fl).

12. Dispositif d'assistance pour une opération endoscopique selon la revendication 1 ou 11,
dans lequel une surface formant le commutateur de fonctionnement est inclinée par rapport à la surface plate (3fl), et l'inclinaison de la surface est plus haute au niveau d'un côté d'extrémité proximale de celle-ci qu'au niveau d'un côté d'extrémité distale de celle-ci.

13. Dispositif d'assistance pour une opération endoscopique selon l'une quelconque des revendications 1, 11 ou 12,
dans lequel le commutateur de fonctionnement (5) et attachable et détachable par rapport au premier élément de poignée (3, 103).

14. Dispositif d'assistance pour une opération endoscopique selon la revendication 1, dans lequel le premier élément de poignée (3, 103) et le deuxième élément de poignée (4, 104) sont raccordés par une partie de raccordement.

15. Dispositif d'assistance pour une opération endoscopique selon la revendication 14,
dans lequel la partie de raccordement est une charnière (9) et décale le premier élément de poignée (3, 103) et le deuxième élément de poignée (4, 104) d'un état ouvert à un état fermé ou d'un état fermé à un état ouvert par l'intermédiaire d'un mouvement de rotation de celle-ci.

16. Dispositif d'assistance pour une opération endoscopique selon la revendication 15,
dans lequel la charnière (9) est une charnière (9) munie d'un ressort.

17. Dispositif d'assistance pour une opération endoscopique selon la revendication 14,
dans lequel la partie de raccordement comprend :
une plaque coulissante (105) d'une forme courbée incluant des trous oblongs (114), et des trous de vis (110) pour fixer solidairement la plaque coulissante (105) sur le premier élément de poignée (103) ;
une plaque (106) de rétention de plaque coulissante comprenant des parties convexes (109) disposées à travers les trous oblongs (114) de la plaque coulissante (105) dans les trous formés dans le deuxième élément de poignée (4, 104) pour attacher de manière coulissante la plaque coulissante (105) sur le deuxième élément de poignée (4, 104) ; et
un ressort de sollicitation (107) prévu sur la plaque (106) de rétention de plaque coulissante pour solliciter le premier élément de poignée (3, 103), auquel la plaque coulissante (105) est fixée solidairement, et
dans lequel, lorsque le premier élément de poignée (3, 103) et le deuxième élément de poignée (4, 104) sont amenés dans un état fermé contre la force de sollicitation du ressort de sollicitation (107), la surface d'ouverture et de fermeture du premier élément de poignée (3, 103) et la surface d'ouverture et de fermeture du deuxième élément de poignée (4, 104) viennent en contact l'une avec l'autre, et lorsque le premier élément de poignée (3, 103) et le deuxième élément de poignée (4, 104) sont amenés dans un état ouvert par la force de sollicitation du ressort de sollicitation (107), la surface d'ouverture et de fermeture du premier élément de poignée (3, 103) et la surface d'ouverture et de fermeture du deuxième élément de poignée (4, 104) sont séparées l'une de l'autre pour être amenées dans un état ouvert.
